# EUROPEAN PATENT APPLICATION

(11) **EP 2 050 387 A1**
(43) Date of publication of application: **22.04.2009**
(21) Application number: 08018066.4
(22) Date of filing: 15.10.2008
(51) Int. Cl.: A61B 5/0245, A61B 5/0452, A61B 5/0432

(54) **Heart-rate variability analysis method and analysis device**

(30) Priority: 18.10.2007 JP 2007271434
(71) Applicant: Nipro Corporation, Kita-ku, Osaka-shi, Osaka-fu, 531-8510 (JP); MHRKS GATE Co., Ltd., Chuo-ku Tokyo 104-0045 (JP)
(72) Inventor: Kamata, Tohru, Chiba-shi, Chiba, 261-0001 (JP)
(74) Representative: Bertsch, Florian Oliver

(57) **Abstract**

The present invention provides a heart-rate variability analysis method and analysis device that have a 24 hour monitoring function, that have a function for the highprecision measuring of the R-R interval heart-rate, and that can exclude the effect of respiratory movement on heart-rate variability. The present invention also provides a heart-rate variability analysis device capable of performing a detailed analysis of the heart rate.
The heart-rate variability analysis device comprises a measurement portion (1) and an analysis display portion (2). The measurement portion (1) has first and second cardiac action potential measuring means (11), (12) that are attached to the thoracic region and the diaphragmatic region, respectively; three-axis acceleration measuring means (13,14).

## Description

### BACKGROUND OF THE INVENTION

### 1. Field of the Invention

The present invention relates to a heart-rate variability analysis method and analysis device which are able to analyze, display and/or print heart-rate variability from a cardiac action potential signal from the thoracic and diaphragmatic regions measured at a high-performance time resolution, and an acceleration signal measured by an accelerometer.

### 2. Description of the Related Art

A Holter electrocardiogram is an examination method for evaluating cardiac function through continuously recording an electrocardiogram of every heartbeat over a prolonged period of time, in normal daily life. Currently, this method is being used as a technique for accurately and precisely extracting abnormalities in cardiovascular function, so as to predict lifestyle-related diseases.
Recently, the existence of fluctuation in heart-rates being measured has been discovered through improvements in the time resolution of Holter monitors. In a patient, measuring the heart-rate variability (fluctuation) is expected to predict and prevent aggravation of e.g., cardiovascular disorders such as sudden death, reduced cardiac function, ventricular remodeling, or stroke; and lifestyle related disease such as diabetes, obesity, hyperlipemia, and high blood pressure. For apparently healthy individuals, it is also hoped that treatment may be provided before symptoms occur.
Also, heart-rate variability is strongly governed by the autonomic nervous system in particular. Therefore, it is hoped that autonomic nerve function can be analogized with sufficient accuracy through analyzing heart-rate variability.

A previously proposed heart-rate variability measuring device for analyzing heart-rate variability circumvents the effects of artifacts and arrhythmia of abnormal intervals on the basis of statistical techniques (Japanese Laid-Open Patent Application No. 5-317277).
The heart-rate variability measuring device has heart-rate signal detecting means, heart-rate interval timing means, sorting means for arranging heart-rate interval data in order of size, heart-rate data analyzing means, and output means for displaying or printing out indices. The heart-rate data analyzing means, for example, sets a middle region using a quartile range method, and computes a quartile range corresponding to the difference between a first quartile and a third quartile as evaluation indices of heart-rate variability.

The time resolution of Holter monitors (ex. 8 msec) has improved over the past several years. However, the time resolution of Holter monitors has still been inadequate, and unable to provide precise verification of the peak position and shape of the R wave. Consequently, R-R intervals cannot be precisely measured by conventional Holter monitors.
Also, variations in the respiratory system are included in heart-rate variability. However, no conventional electrocardiograph has been able to exclude the effect of the lungs (the effect of respiratory movement) from the heart-rate variability. Consequently, in cases in which an attempt is made to exclude the effect of the lungs from the heart-rate variability, hospitalization and complicated examinations have been necessary.

### SUMMARY OF THE INVENTION

In view of the abovementioned circumstances it is an object of the present invention to provide a heart-rate variability analysis method and analysis device that has a 24 hour electrocardiograph function and a high-precision R-R interval heart-rate measuring function, and that can exclude the effect of respiratory movement on heart-rate variability.
Also, it is an object of the present invention to provide heart-rate variability analysis device that can analysis the heart-rate variability in detail.

The heart-rate variability analysis method of the present invention is characterized by analyzing heart-rate variability on the basis of a cardiac action potential signal measured from the thoracic region at a time resolution of 3 msec or less, a cardiac action potential signal measured from the diaphragmatic region at a time resolution of 3 msec or less, and an acceleration signal of the body. It is preferable that the following six categories of data are calculated from the analysis of the cardiac action potential signal and displayed, in order to readily diagnose illness (including presymptomatic illness) from the analysis data. The six categories of data are instantaneous heart-rate time-series data, heart-rate variability rhythm ratio data, heart-rate variability fluctuation frequency HF component time-series data, heart-rate variability fluctuation frequency HF component rhythm ratio data, heart-rate variability fluctuation frequency LF/HF component time-series data, and heart-rate variability fluctuation frequency LF/HF component rhythm ratio data. Taking into consideration ease of use and cost, it's preferable that the cardiac action signal is measured by a Holter monitor having a time resolution of 0.1 to 3.0 msec.

The heart-rate variability analysis device of the present invention includes a measurement portion and an analysis display portion. The measurement portion includes first cardiac action potential measuring means, second cardiac action potential measuring means, and three-axis acceleration measuring means.
The first cardiac action potential measuring means measures a cardiac action potential signal from the thoracic region with a time resolution of 3 msec or less. The second cardiac action potential measuring means measures a cardiac action potential signal from the diaphragmatic region with a time resolution of 3 msec or less. The three-axis acceleration measuring means measures an acceleration signal. The analysis display portion receives the cardiac action potential signal and the acceleration signal transmitted from the measurement portion, and analyzes and displays heart-rate variability. It's preferable that six categories of data are obtained through analysis of the cardiac action potential signal and are displayed. The six categories of data are instantaneous heart-rate time-series data, heart-rate variability rhythm ratio data, heart-rate variability fluctuation frequency HF component time-series data, heart-rate variability fluctuation frequency HF component rhythm ratio data, heart-rate variability fluctuation frequency LF/HF component time-series data, and heart-rate variability fluctuation frequency LF/HF component rhythm ratio data. Moreover, it's preferable that the cardiac action potential signal is measured by a Holter monitor having a time resolution of 0.1 to 3.0 msec. If necessary, an inclinometer may be incorporated in the Holter monitor.
The present invention has been described in general above. However, a greater understanding can be obtained through referencing several specific examples. The examples given in the present disclosure are merely provided for illustrative purposes, and the present invention shall not be construed to be limited thereto.

According to the present invention, the following effects can be expected. Specifically, heart-rate variability includes aspect of the heart system and aspect of the respiratory system. The heart-rate variability analysis device of the present invention is configured to be able to measure an acceleration signal from two places; i.e., the thorax and the diaphragm (upper abdomen), in order to precisely ascertain respiratory movement. Consequently, the effect of the lungs (the effect of respiratory movement) can be eliminated. Moreover, attacks of apnea during sleep are detected through the use of the accelerometer, and respiratory movement can be physically measured.
Also, according to the present invention, cardiac action potential is measured at a high time resolution of 3 msec or less. Therefore, a detailed heart-rate variability analysis is possible. Moreover, the heart-rate variability analysis device of the present invention is configured to be able to output necessary data as a report in the form of a graph or another format. Therefore, the data can be used as a method for objectively assessing the state of autonomic nerve function, which has until now been assessed only through a mixture of subjective presumptions and trial-and-error.

### BRIEF DESCRIPTION OF THE DRAWINGS

FIG. 1 is a block diagram of the heart-rate variability device of the present invention;
FIG. 2 is a diagram illustrating a Holter monitor of the present invention;
FIG. 3 is a diagram showing a housing in which a receiver and interface shown in FIG. 1 are incorporated;
FIG. 4 is a flow chart showing a heart-rate variability analysis program;
FIG. 5 is a flow chart showing a heart-rate variability analysis program;
FIG. 6 is a flow chart showing a heart-rate variability analysis program;
FIG. 7 is an example of instantaneous heart-rate time-series data;
FIG. 8 is an example of heart-rate variability fluctuation frequency HF component time-series data;
FIG. 9 is an example of heart-rate variability fluctuation frequency LF/HF component time-series data;
FIG. 10 is an example of heart-rate variability rhythm ratio data;
FIG. 11 is an example of heart-rate variability fluctuation frequency HF component rhythm ratio data; and
FIG. 12 is an example of heart-rate variability fluctuation frequency LF/HF component rhythm ratio data.
   KEY

- 1: Measurement portion
- 11: First cardiac action potential measuring means
- 12: Second cardiac action potential measuring means
- 13, 14: Three-axis acceleration measuring means (three-axis accelerometer)
- 15, 16: Transmitter
- H1, H2: Holter monitor
- 111, 121: Housing
- 112, 122: Electrocardiographic circuit
- 113, 123: Electrode
- 114, 124: Indicator for checking operation
- 2: Analysis display portion
- 21: Receiver
- 211: Housing
- 212: Switch
- 213: Indicator for checking operation
- 22: Interface
- 23: Computer

### DESCRIPTION OF THE PREFERRED EMBODIMENTS

The heart-rate variability analysis device of the present invention includes a Holter monitor and an analysis display portion. The Holter monitor is comprised of a first cardiac action potential recorder and a second cardiac action potential recorder. The first cardiac action potential recorder has first cardiac action potential measuring means, a three-axis accelerometer, and a transmitter. The first cardiac action potential measuring means measures a cardiac action potential signal from the thoracic region with a 1 msec time resolution. The three-axis accelerometer measures an acceleration signal. The second cardiac action potential recorder has second cardiac action potential measuring means, a three-axis accelerometer, and a transmitter. The second cardiac action potential measuring means measures a cardiac action potential signal from the diaphragmatic region with a 1 msec time resolution. The three-axis accelerometer measures an acceleration signal. The analysis display portion receives the cardiac action potential signal and acceleration signal transmitted from the Holter monitor, and analyzes and displays the heart-rate variability. Data calculated by an analysis is divided into six categories and each data of the six categories is displayed on the heart-rate variability analysis device of the present invention. Each data of six categories is instantaneous heart-rate time-series data, heart-rate variability rhythm ratio data, heart-rate variability fluctuation frequency HF component time-series data, heart-rate variability fluctuation frequency HF component rhythm ratio data, heart-rate variability fluctuation frequency LF/HF component time-series data, and heart-rate variability fluctuation frequency LF/HF component rhythm ratio data.

### [First embodiment]

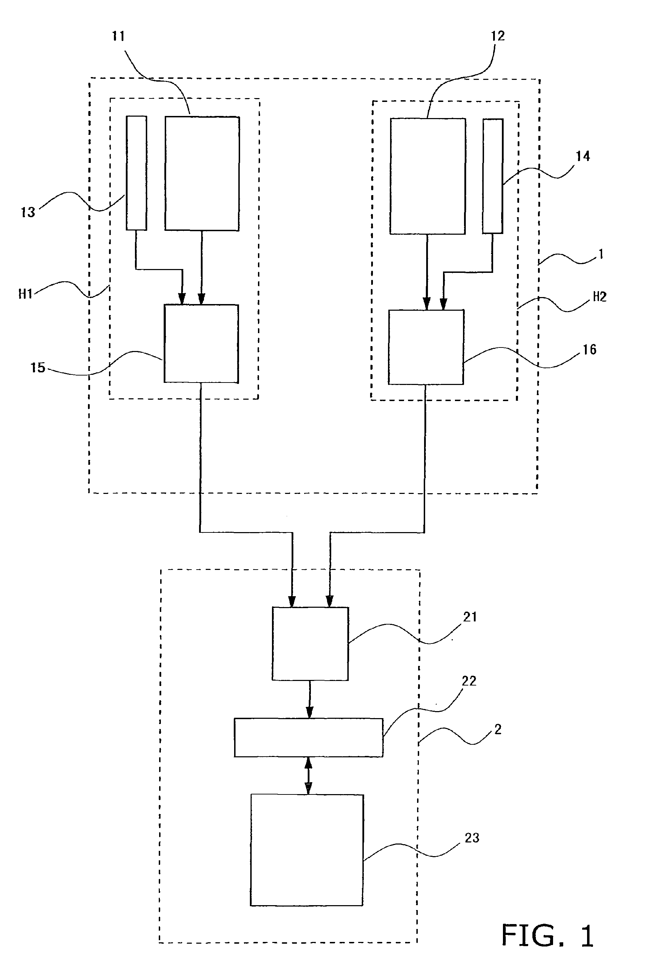

First embodiment will be illustrated with reference to FIGS. 1 to 3.

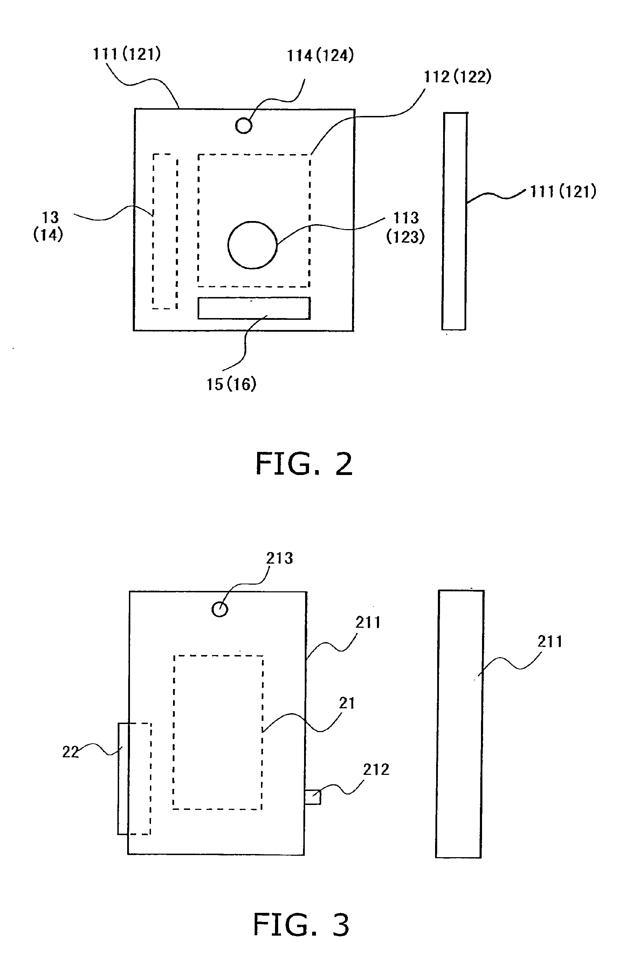

FIG. 1 is a block diagram of the heart-rate variability device of the present invention. FIG. 2 is a diagram of the Holter monitor of the present invention. FIG. 3 is a diagram showing a housing in which a receiver and interface shown in FIG. 1 are incorporated.
As shown in FIG 1, the heart-rate variability analysis device of the first embodiment is comprised of a measurement portion 1 and an analysis display portion 2. The measurement portion 1 has first cardiac action potential measuring means 11 that is attached to a thoracic region, second cardiac action potential measuring means 12 that is attached to a diaphragmatic region, three-axis acceleration measuring means 13, 14; and transmitters 15, 16. The transmitters 15, 16 transmit to the analysis display portion 2 a cardiac action potential signal measured by the cardiac action potential measuring means 11, an acceleration signal measured by the acceleration measuring means 13, a cardiac action potential signal measured by the cardiac action potential measuring means 12, and an acceleration signal measured by the acceleration measuring means 14. The analysis display portion 2 includes a receiver 21, an interface 22, and a computer 23. The receiver 21 receives the cardiac action potential signal and the acceleration signal transmitted from the transmitters 15, 16. The interface 22 transmits the signal received by the receiver 21 to the computer 23. The computer 23 analyzes and displays and/or prints the heart-rate variability on the basis of the cardiac action potential signal and the acceleration signal received via the interface 22.

The first and second cardiac action potential measuring means 11, 12 have a time resolution of 3 msec or less. The peak of the R wave of the cardiac action potential signal measured by the first and second cardiac action potential measuring means 11, 12 is automatically recognized using an R wave recognition algorithm. The R-R interval time series is subjected to an A/D conversion at a time resolution of a sampling interval of 3 msec or less. The time series data (raw data) that has been subjected to the A/D conversion is transmitted to the analysis display portion 2. As shown in FIG. 2, Holter monitor H1, H2 employed in measuring the cardiac action potential signal has a housing 111 (121), an electrocardiographic circuit 112 (122) incorporated in the housing 111 (121), an electrode 113 (123), a three-axis accelerometer (three-axis acceleration measuring means) 13 (14), and a transmitter 15 (16) incorporated in the housing 111 (121). The cardiac action potential signal and acceleration signal measured by the Holter monitor H1, H2 are transmitted to the analysis display portion 2 by the transmitter 15 (16). In FIG. 2, numbers 114 (124) is a reference number which shows an indicator for checking operations, and number 113 (123) is a reference number which shows an electrode. The electrode 113 (123) and the electrocardiographic circuit 112 (122) comprise the cardiac action potential measuring means 11 (12).

Holter monitor H1, H2 has a time resolution of 0.1 to 3 msec. The Holter monitor H1, H2 is miniaturized and is able to be attached to the skin using an electrode seal or the like (not shown). Moreover, when waterproof electrode seals are used, bathing is possible, and the cardiac action potential can be measured over a 24 hour period. Any type of monitor having a time resolution of 0.1 to 3 msec can be employed; however, the monitor is preferably of a size and shape allowing attachment to the skin. In case that a monitor is designed to have a time resolution of 0.1 msec or less, the device becomes too large to be practical. In case that a monitor is designed to have a time resolution of 3 msec or above, the position of the peak of the R wave, which is used for measuring the heart-rate interval (R-R interval), is indistinct, and heart-rate variability cannot be precisely measured. A piezoelectric acceleration sensor is employed as the three-axis accelerometer 13, 14. The accelerometer does not necessarily have to be incorporated in the monitor 11, 12. A 2.4 GHz band data communication system, which has an advanced power saving function and is comprised of ceramic module, is employed as the transmitter 15. TDMA (Time Division Multiple Access) is employed as the transmission method.

The analysis display portion 2 includes the receiver 21, the interface 22, and the computer 23. The receiver 21 receives the cardiac action potential signal and the acceleration signal transmitted from the measurement portion 1. The interface 22 links the cardiac action potential signal and the acceleration signal received by the receiver 21 to the computer 23. As shown in FIG. 3, the receiver 21 is housed in the housing 211 along with the interface 22. The receiver 21 is driven by a switch 212 being turned on. The received signals are stored in a storage medium of the interface 22, and are transmitted to the computer 23 as needed. Number 213 is a reference number which shows an indicator for checking operations. A 2.4 GHz band data communication system, which has an advanced power saving function and is comprised of ceramic module, is employed in the receiver 21. Furthermore, TDMA (Time Division Multiple Access) is employed as the transmission method of the receiver 21. A program for analyzing heart-rate variability on the basis of the cardiac action potential signal and the acceleration signal from the thoracic and diaphragmatic regions is installed on the computer 23. As heart-rate variability that is analyzed by the program, six categories of data are displayed and printed. The six categories of data are instantaneous heart-rate time-series data, heart-rate variability rhythm ratio data, heart-rate variability fluctuation frequency HF component time-series data, heart-rate variability fluctuation frequency HF component rhythm ratio data, heart-rate variability fluctuation frequency LF/HF component time-series data, and heart-rate variability fluctuation frequency LF/HF component rhythm ratio data.

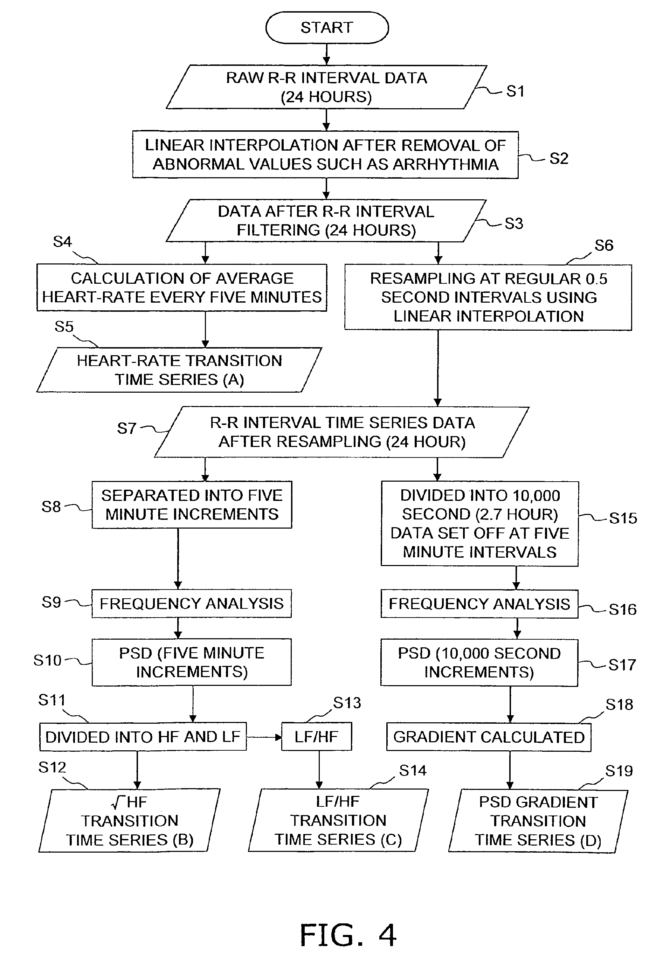

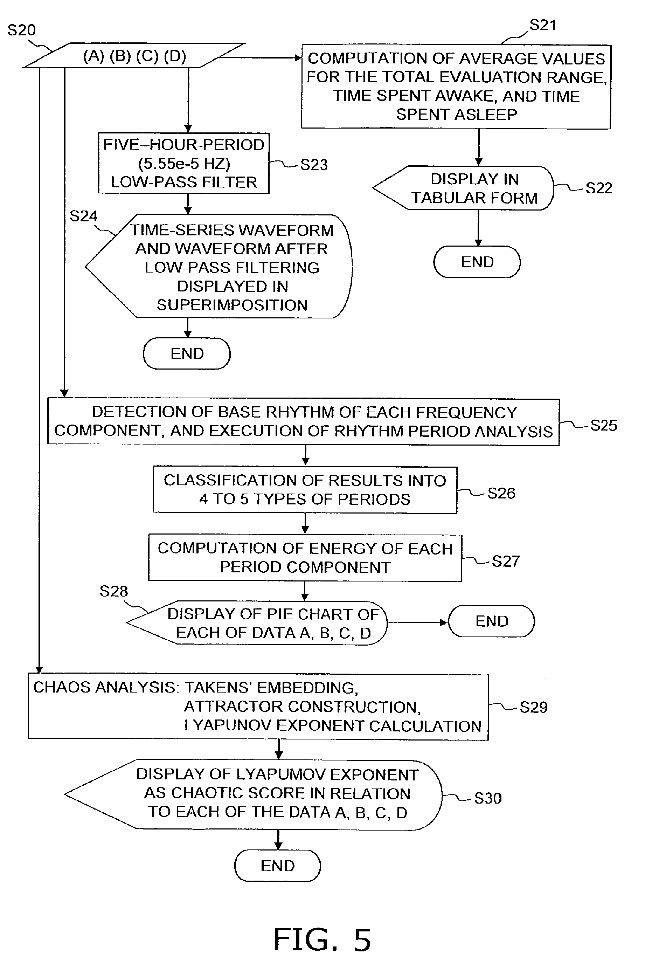

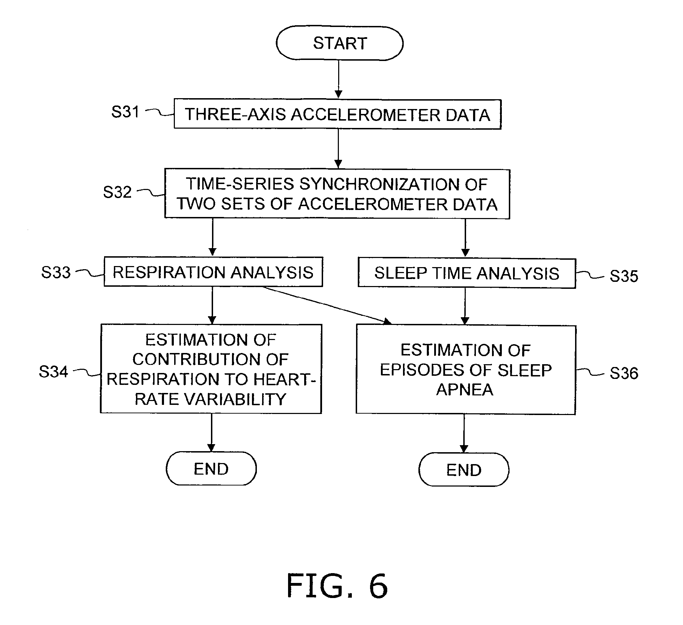

FIGS. 4, 5, and 6 are flow charts showing the heart-rate variability analysis program. When raw R-R interval data (24 hour) is entered into the computer 23 (S1), first, linear interpolation is performed after arrhythmia and other abnormal values have been removed (S2), and post-R-R interval filter data (24 hour) is output (S3). Calculating the average heart-rate every five minutes (S4) allows the post-filter data to be output as a heart-rate transition time series (A) (S5). Resampling is performed at regular 0.5 second intervals using linear interpolation (S6), and the data is output as post-resampling R-R interval time series data (24 hour) (S7). The post-resampling data is separated into five minute increments (S8), and then subjected to a frequency analysis (S9), output as power spectral density (PSD) for each five minute increment (S10), and divided into 0.15 to 0.4 Hz power (HF) and 0.04 to 0.15 Hz power (LF) (S11). The square root of the HF is determined, and the √HF transition time series (B) is output (S12). The LF is divided by the HF (S13), and the LF/HF transition time series (C) is output (S14). The post-resampling data output in S7 is divided into 10,000 second (2.7 hour) data which is shifted at five minute intervals (S15). The 10,000 second (2.7 hour) data is then subjected to a frequency analysis (S16), and the results of the frequency analysis are output as a power spectral density (PSD) for a 10,000-second interval (S17). The slope of the power spectral density is then calculated (S18), and the PSD slope transition time series (D) is output (S19).

Next, average values of total evaluation range, awake time range, and asleep time range are computed on the basis of the data A, B, C, D (S20, S21), and the average values of the total evaluation range, awake time range, and asleep time range are displayed in tabular form (S22). The data A, B, C, D (S20) is separately passed through low-pass filter with a five-hour-period (5.55e-5 Hz) (S23), and the time-series waveform and the waveform after low-pass filtering are displayed in superimposition (S24). In addition, the base rhythm of each of the frequency components of the data A, B, C, D (S20) is detected, and a rhythm period analysis is performed (S25). The results are classified into 4 to 5 types of periods (S26), and the amount of energy of each period component is computed (S27). The percentage of each component in relation to each of the data A, B, C, D is then calculated, and the results are displayed in a pie chart (S28). Alternatively, a chaos analysis (Takens' embedding, attractor construction, and Lyapunov exponent calculations) of the data A, B, C, D (S20) is also performed (S29). The Lyapunov exponent is then displayed as a chaotic score in relation to each of the data A, B, C, D (S30).

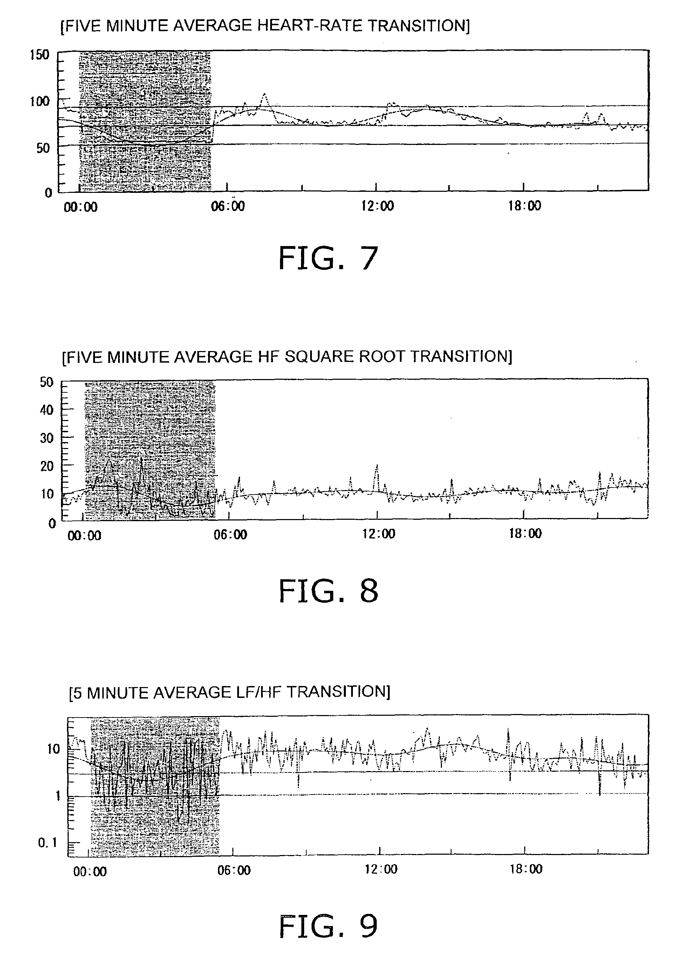

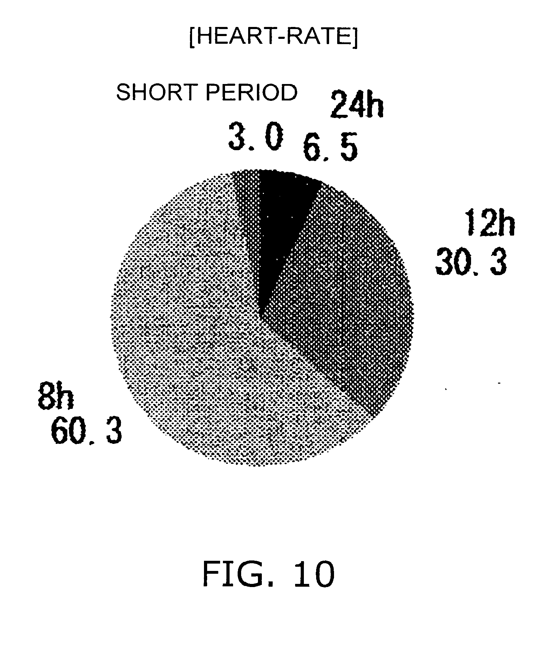

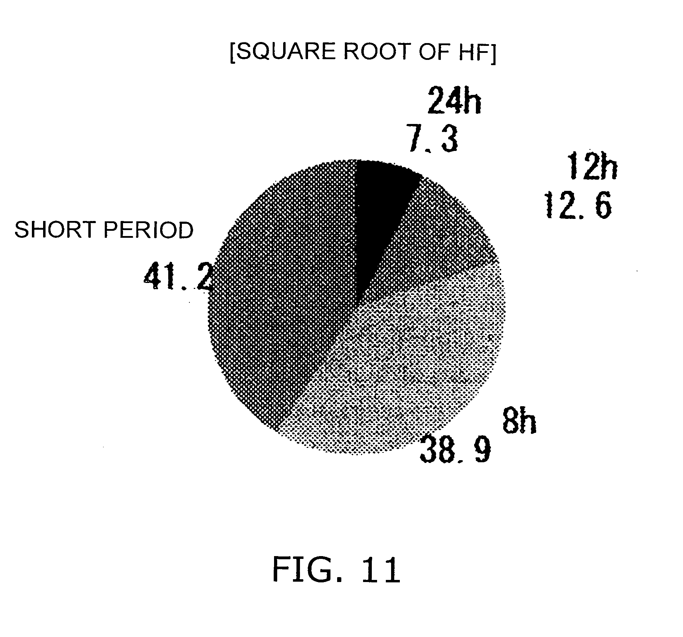

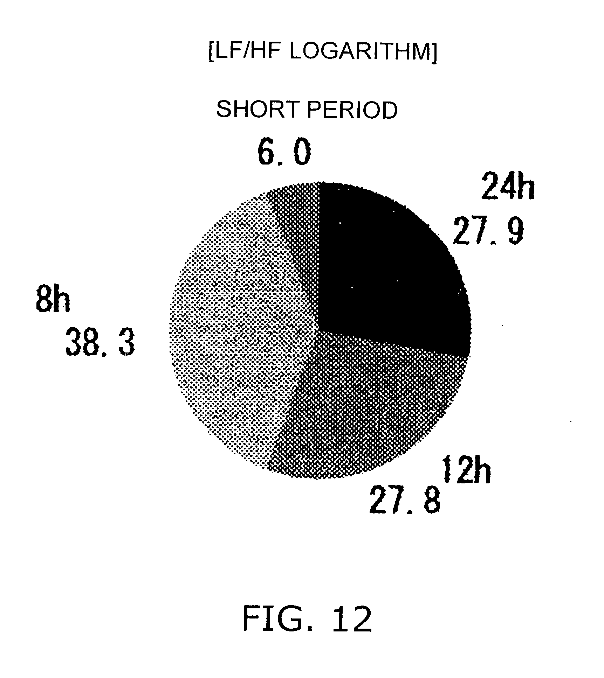

The data A, B, C displayed as a waveform in S24 are, respectively, instantaneous heart-rate time-series data, heart-rate variability fluctuation frequency HF component time-series data, and heart-rate variability fluctuation frequency LF/HF component time-series data. Examples of the waveform charts are shown in FIGS. 7 to 9. The data A, B, C displayed in a pie chart in S28 are, respectively, heart-rate variability rhythm ratio data, heart-rate variability fluctuation frequency HF component rhythm ratio data, and heart-rate variability fluctuation frequency LF/HF component rhythm ratio data. Examples of the pie charts are shown in FIGS. 10 to 12.
The presence or absence of arrhythmia and cardiac palpitation symptoms is inferred from the instantaneous heart-rate time-series data. The time series transition of the parasympathetic nerve activity is inferred from the heart-rate variability fluctuation frequency HF component time-series data. The time series transition of the sympathetic nerve activity is inferred from the heart-rate variability fluctuation frequency LF/HF component time-series data. The stability for the activity of the cardiopulmonary function is inferred from the heart-rate variability rhythm ratio data. The stability for the activity of the parasympathetic nerve is inferred from the heart-rate variability fluctuation frequency HF component rhythm ratio data. The stability for the activity of the sympathetic nerve is inferred from the heart-rate variability fluctuation frequency LF/HF component rhythm ratio data.

When the acceleration signal (three-axis accelerometer data) is entered into the computer 23 (S31), the two sets of accelerometer data are synchronized in the time series (S32). Respiration analysis is then performed (S33), and the contribution of respiration to heart-rate variability is inferred (S34). When the two sets of accelerometer data are synchronized in the time series (S32), a sleep time analysis is performed (S35), and apnea attacks during sleep are inferred (S36).

## Claims

1. A heart-rate variability analysis method for analyzing heart-rate variability on the basis of a cardiac action potential signal measured from the thoracic region at a time resolution of 3 msec or less, a cardiac action potential signal measured from the diaphragmatic region at a time resolution of 3 msec or less, and an acceleration signal of the body.

2. The heart-rate variability analysis method as recited in Claim 1, wherein
six categories of data, which are instantaneous heart-rate time-series data, heart-rate variability rhythm ratio data, heart-rate variability fluctuation frequency HF component time-series data, heart-rate variability fluctuation frequency HF component rhythm ratio data, heart-rate variability fluctuation frequency LF/HF component time-series data, and heart-rate variability fluctuation frequency LF/HF component rhythm ratio data, are calculated on the basis of analysis of the cardiac action potential signal, and displayed.

3. The heart-rate variability analysis method as recited in Claim 1 or Claim 2, wherein
the cardiac action potential signal is measured by a Holter monitor which has a time resolution of 0.1 to 3 msec.

4. A heart-rate variability analysis device comprising:
a measurement portion (1) including first cardiac action potential measuring means (11) for measuring a cardiac action potential signal from the thoracic region, the first cardiac action potential measuring means having a time resolution of 3 msec or less; second cardiac action potential measuring means (12) for measuring a cardiac action potential signal from the diaphragmatic region, the second cardiac action potential measuring means having a time resolution of 3 msec or less; and three-axis acceleration measuring means (13) for measuring an acceleration signal; and
an analysis display portion (2) for receiving the cardiac action potential signal and the acceleration signal transmitted from the measurement portion, and analyzing and displaying heart-rate variability.

5. The heart-rate variability analysis device as recited in Claim 4, wherein
six categories of data, which are instantaneous heart-rate time-series data, heart-rate variability rhythm ratio data, heart-rate variability fluctuation frequency HF component time-series data, heart-rate variability fluctuation frequency HF component rhythm ratio data, heart-rate variability fluctuation frequency LF/HF component time-series data, and heart-rate variability fluctuation frequency LF/HF component rhythm ratio data, are calculated on the basis of analysis of the cardiac action potential signal, and displayed.

6. The device as recited in Claim 4 or Claim 5, wherein a cardiac action potential signal is measured using a Holter monitor which has a time resolution of 0.1 to 3 msec.
